# EUROPÄISCHE PATENTANMELDUNG

(11) **EP 1 782 782 A2**
(43) Veröffentlichungstag der Anmeldung: **09.05.2007**
(21) Anmeldenummer: 06021995.3
(22) Anmeldetag: 20.10.2006
(51) Int. Cl.: A61F 13/00, A61F 13/15

(54) **Verfahren zum Herstellen von Flächengebilden zur Wundversorgung**

(30) Priorität: 04.11.2005 DE 102005053149
(71) Anmelder: Paul Hartmann AG, 89522 Heidenheim (DE)
(72) Erfinder: Effing, Jochem, Dr., 65779 Kelkheim-Fischbach (DE); Halbauer, Rainer, 73569 Obergröningen (DE)
(74) Vertreter: Dreiss, Fuhlendorf, Steimle & Becker

(57) **Zusammenfassung**

Die Erfindung betrifft ein Verfahren zum Herstellen von Flächengebilden aus Vliesmaterial zur Wundversorgung, insbesondere Kompressen, bei dem ein erster Teil einer Flachmaterialbahn zunächst entlang einer parallel zu einer Maschinenrichtung verlaufenden ersten Faltkante so umgelegt wird, dass der erste Teil der Flachmaterialbahn den übrigen anderen Teil zumindest teilweise zur Bildung einer zweiten Lage überdeckt, bei dem anschließend mindestens ein weiteres Mal ein weiterer Teil der Flachmaterialbahn entlang einer parallel zu der Maschinenrichtung verlaufenden weiteren Faltkante so umgelegt wird, dass der weitere Teil wiederum auf dem übrigen anderen Teil der Flachmaterialbahn zu liegen kommt, unter Ausbildung eines zumindest teilweise wenigstens dreilagigen Flächengebildes, wobei sämtliche Faltkanten des erhaltenen Flächengebildes parallel zur Maschinenrichtung verlaufen und das Flächengebilde eine die Ober- sowie eine die Unterseite bildende Lage aufweist, wobei sich diese Lagen wenigstens über 85% der Breite des Flächengebildes erstrecken sowie ein Flächengebilde insbesondere eine Kompresse.

## Beschreibung

Die Erfindung betrifft ein Verfahren zum Herstellen von Flächengebilden aus Vliesmaterial zur Wundversorgung, insbesondere Kompressen, bei dem ein erster Teil einer Flachmaterialbahn zunächst entlang einer parallel zu einer Maschinenrichtung verlaufenden ersten Faltkante so umgelegt wird, dass der erste Teil der Flachmaterialbahn einen übrigen anderen Teil zumindest teilweise zur Bildung einer zweiten Lage überdeckt sowie ein entsprechendes Flächengebilde.

Derartige Flächengebilde zur Wundversorgung, insbesondere Kompressen, sind im Stand der Technik vielfach bekannt. Sie werden zumeist aus einem Vliesmaterial oder aber auch aus einem Gazematerial oder ähnlichem hergestellt.

So beschreibt beispielsweise die WO 2004/103872 A1 eine Vorrichtung zum Herstellen mehrlagiger Gegenstände, wie insbesondere Kompressen, Verbandmaterial, Tupfer oder dergleichen, wobei hier längs einer Transportbahn, über die die Materialabschnitte transportiert werden, eine Faltstation angeordnet ist, die aus dem Materialabschnitt durch Umschlagen entlang einer Linie eine Falte bildet, so dass dann zwei Teile des Materialabschnitts übereinander liegen und dadurch eine zweilagige Ausbildung des gefalteten Gegenstands entsteht. Dabei soll vorgesehen sein, dass die Faltstation quer zur Transportrichtung verlaufende Falten herstellt. Insbesondere sollen sowohl Quer- als auch Längsfalten hergestellt erzeugt werden.

Darüber hinaus ist es beispielsweise aus DE 28 38 590 Al bekannt, Wundkompressen mittels einer Vorrichtung herzustellen, wobei auch hier ein Mullstreifen zunächst quer und dann mittels einer Längsfalteinrichtung in Längsrichtung gefaltet wird. Hierzu wird der Mullstreifen vorab geschnitten und jede einzelne Kompresse den verschiedenen Faltvorgängen unterworfen.

Darüber hinaus ist aus der DE 35 35 539 A1 eine Vorrichtung zum Herstellen von Wundkompressen bekannt, wobei hier zunächst eine Ablängung erfolgt und dann über eine Querfalteinrichtung die erzeugten Stücke dreimal quer gefaltet und ein- oder mehrfach danach durch eine Längsfalteinrichtung längsgefaltet werden.

Weitere Kompressen, die sowohl Längs- als auch Querfaltungen aufweisen und damit in herkömmlicher Weise hergestellt sind, lassen sich auch der EP 0 371 893 A1 sowie der EP 0 242 415 B1 entnehmen.

Darüber hinaus ist ein Verfahren zum Herstellen einer Kompresse für die operative und stationäre Wundbehandlung mit einer Wattefüllung in einer Gazehülle auch aus der DE 34 14 737 A1 bekannt, bei der eine solche Faltung vorgenommen wird, dass die Wattepolster vollständig in der Gazehülle eingeschlossen sind mittels Längs- und Querfaltung.

Nachteilig bei sämtlichen der im Stand der Technik bekannten Verfahren zum Herstellen von Flächengebilden zur Wundversorgung, insbesondere von Kompressen, ist, dass jede einzelne Wundkompresse den Faltvorgängen unterzogen werden muss.

Hierdurch wird die Verfahrensökonomie stark beeinträchtigt Darüber hinaus gibt es eine Vielzahl von Wundauflagen, die lediglich eine Seite aufweisen, die zum Auflegen auf die Haut vorgesehen sind.

Die Erfindung stellt sich nun die Aufgabe, ein Verfahren zum Herstellen von Flächengebilden zur Wundversorgung bereitzustellen, sowie auch ein entsprechendes Produkt, bei dessen Herstellung bei gleichbleibend guter Produktqualität die vorstehend genannten Nachteile vermieden werden können und das darüber hinaus beidseitig verwendbar ist.

Die Erfindung löst diese Aufgabe durch ein gattungsgemäßes Verfahren zum Herstellen von Flächengebilden zur Wundversorgung, insbesondere Kompressen, bei dem anschließend mindestens ein weiteres Mal ein weiterer Teil der Flachmaterialbahn entlang einer parallel zu der Maschinenrichtung verlaufenden weiteren Faltkante so umgelegt wird, dass der weitere Teil wiederum auf einem übrigen anderen Teil der Flachmaterialbahn zu liegen kommt, unter Ausbildung eines zumindest teilweise wenigstens dreilagigen Flächengebildes, wobei sämtliche Faltkanten des erhaltenen Flächengebildes parallel zur Maschinenrichtung angeordnet sind und das Flächengebilde eine die Ober- sowie eine die Unterseite bildende Lage aufweist, wobei sich diese Lagen wenigstens über 85% der Breite des Flächengebildes erstrecken.

Die Dreilagigkeit kann dabei nur abschnittsweise erreicht werden, wird jedoch vorzugsweise über die gesamte Breite des Bahnmaterials erzielt. Um eine beidseitige Verwendbarkeit des Flächengebildes sicherzustellen, sind diese Ober- und Unterseiten vorzugsweise vollflächig ausgebildet; erstrecken sich jedoch aber wenigstens über 85% der Breite des Flächengebildes.

Auf die vorstehend beschriebene Weise kann erreicht werden, dass eine Kompresse in annähernd beliebiger Lagenstärke hergestellt werden kann, wobei vor dem Faltvorgang keine Ablängung der Flachmaterialbahn erfolgen muss, sondern der Faltvorgang kontinuierlich oder quasikontinuierlich erfolgen kann. Erst nach sämtlichen Faltvorgängen muss eine Ablängung auf die gewünschte Kompressengröße erfolgen. Auf diese Weise kann insbesondere auch bei verschiedenen Kompressen bzw. textilen Flächengebilden insbesondere verschiedener Größe die Herstellung vereinfacht und Umrüstzeiten vermieden werden. Die Faltung ist beim vorliegenden Verfahren vollständig unabhängig von der späteren Größe der Kompresse gemessen in Längsrichtung der Flachmaterialbahn.

Durch dieses "kontinuierliche" oder "quasi-kontinuierliche" Verfahren kann die Produktionseffektivität deutlich gesteigert werden.

Dabei können sich die Lagen jeweils vorteilhaft über die gesamte Breite erstrecken, wobei eine Lage auch durch zwei umgelegte Teile gebildet sein kann, die aufeinander zu weisen. Die beiden aufeinander zuweisenden Teile müssen sich dabei in einer Ebene befinden, um eine Lage zu definieren. Dabei wird eine Oberlappung der aufeinander zuweisenden Teile voneinander von weniger als 5 insbesondere weniger als 2 % und insbesondere von 0 der Breite des fertigen Flächengebildes auch noch als in einer Ebene liegend angesehen. Bei einer grösseren Oberlappung liegen die beiden Teile nicht mehr in einer Ebene, sondern bilden verschiedene Lagen.

Bevorzugt erstrecken sich die Lagen auf der Ober- und Unterseite über mehr als 85% der Breite, nämlich vorzugsweise 90%, besonders bevorzugt 95% und insbesondere bevorzugt über 100%.

Besonders vorteilhaft ist es dabei, wenn der weitere Teil, der beim zweiten oder weiteren Umlegevorgängen umgelegt wird, nach der vorangehenden Faltung bereits mehrlagig ist. Alternativ kann dieser Teil jedoch auch einlagig sein, insbesondere wenn das Umlegen des ersten Teils lediglich so weit erfolgte, dass er den übrigen Teil nur über eine gewisse Breite (die quer zur Maschinenrichtung gemessen wird) überdeckt.

Vliesmaterialien besitzen dabei durch ihren Herstellungsprozess eine Maschinen- oder Vorzugsrichtung sowie eine Querrichtung dazu. Es ist nun besonders vorteilhaft, wenn die Maschinenrichtung des Vlieses mit der Maschinen- oder Transportrichtung des Verfahrens zusammenfällt.

Besonders bevorzugt ist es, das Verfahren zur Bildung von mindestens vier, aber auch mehr als vierlagigen Flächengebilden einzusetzen, wobei sämtliche Faltungen parallel zur Maschinenrichtung vorgenommen werden.

Darüber hinaus ist ein Verfahren bevorzugt, bei dem mindestens drei Faltkanten gebildet werden, wobei sämtliche Faltkanten parallel zur Maschinenrichtung verlaufen.

Dabei ist es besonders vorteilhaft, wenn der jeweilige umgelegte Teil den übrigen Teil um mindestens ein Viertel, ein Drittel oder vorzugsweise um mindestens die Hälfte und insbesondere ganz überdeckt. Die Überdeckung wird hierbei stets in Maschinenquerrichtung betrachtet.

Nach der Faltung der Flächengebilde kann dann quer zur Richtung der Faltkanten und damit auch quer zur Maschinenrichtung ein Schnitt- oder Ablängvorgang stattfinden, bei dem die fertigen Flächengebilde entsprechend einer gewünschten Länge erzeugt werden.

Nach oder während oder durch diesen Schnitt- oder Ablängvorgang kann ein Verschließen der Schnittkanten oder Verfestigen derselben oder ein weiterer Verfestigungsvorgang der oder des gesamten Flächengebilde vorgenommen werden. Alternativ oder zusätzlich kann weiterhin vorgesehen sein, dass vor dem Ablängvorgang jedoch vorzugsweise nach allen Faltungen ein Verfestigungsvorgang der oder des gesamten Flächengebilde vorgenommen wird.

Das Verschließen der Schnittkanten kann beispielsweise mittels Prägen oder Schweißen sowie ähnlicher Methoden, die im Stand der Technik bekannt sind, erfolgen, so dass gegebenenfalls innen liegende Vliese keinen direkten Wundkontakt besitzen.

Die äussere Vlieslage kann vorzugsweise mit einer oder mehreren Lagen verbunden sein, beispielsweise durch verkleben, verschweissen oder verpressen.

Darüber hinaus können mehrere Vliese mit unterschiedlichen Eigenschaften in Form von mehrschichtigen Materialien oder Laminaten, wobei die einzelnen Schichten miteinander verbunden oder nicht verbunden sein können, verarbeitet werden.

Die Faltung der verschiedenen Schichten kann zusammen erfolgen.

Alternativ können auch sogenannte Funktionsschichten, wie beispielsweise umfassend Aktivkohle zur Geruchsabsorption oder Superabsorber für verbesserte Flüssigkeitsaufnahme, oder andere Schichten vorgesehen sein. Diese können sich auch lediglich über einen Teil der Breite und/oder Länge des Bandmaterials erstrecken. So kann z. B. vorgesehen sein, dass diese dann durch die Faltung z. B. vollständig von einem oder mehreren anderen Vliesen eingeschlossen werden. Auf diese

Weise kann erreicht werden, dass nach Verschließen der Schnittkanten die innen liegenden sogenannten "Funktionsvliese" oder auch andere Materialien wie Superabsorber keinen direkten Wundkontakt mehr haben. Zusätzliche Materialien können dabei auch vor einem Faltvorgang auf die zu faltende Materialbahn aufgebracht, z. B. beschichtet werden, wobei das Aufbringen nicht vollflächig erfolgen muss.

Die obengenannten Funktionsschichten können, müssen aber nicht zusammen mit dem Bahnmaterial gefaltet werden. Es können optional auch nur einige Faltschritte hiermit erfolgen. Auf diese Weise können funktionelle Stoffe in das Flächengebilde integriert werden.

Darüber hinaus muss das Bahnmaterial eine ausreichende Maschinengängigkeit aufweisen sowie eine ausreichende Biegsamkeit in Längsrichtung, um eine Faltung zu ermöglichen. Des Weiteren soll die Zugfestigkeit in Längsrichtung so gestaltet sein, dass ein Abreißen des Materials beim Falten verhindert wird und darüber hinaus muss eine gewisse plastische Verformbarkeit gegeben sein, so dass die Faltung erhalten bleibt.

Beispielsweise kann das Verfahren mit einer Maschine der Firma Mabotex (Staint-Etienne, Frankreich) z. B. der Maschine P100 oder P130 durchgeführt werden, die sogenannte Umlenkbleche zur Erzielung der Faltung aufweisen. Beim Fördern des Bahnmaterials in Längsrichtung wird das Material im Bereich der Umlenkbleche so geführt, dass es in Richtung auf das übrige Bahnmaterial umgelenkt und umgelegt und damit gefaltet wird. Als Umlenkeinrichtung oder Umlenkblech dient in der Regel ein gebogenes Blech, über das bei Bewegung in Maschinenrichtung ein Teil des Bahnmaterials geführt wird und durch die Form des Blechs bei weiterer Bewegung in Längsrichtung der Materialbahn auf das übrige Bahnmaterial kippt.

Durch das vorliegende Verfahren kann die Produktion deutlich beschleunigt werden und darüber hinaus kann das Ablängen und Schneiden an beliebiger Stelle des Bahnmaterials erfolgen, so dass auch die Umrüstzeiten der Maschine verringert werden können. Das Ablängen kann mit üblichen Schneideinrichtungen erfolgen, wie z.B. mit solchen der Firma Mabotex (Staint-Etienne, Frankreich).

Des Weiteren umfasst die Erfindung ein ein Vliesmaterial umfassendes Flächengebilde, insbesondere eine Kompresse zur Wundversorgung, die insbesondere nach dem vorstehend beschriebenen Verfahren hergestellt ist, bei dem sämtliche Faltkanten parallel verlaufen und das zumindest teilweise mindestens dreilagig ausgebildet ist, wobei sich die eine Ober- und eine Unterseite bildenden Lagen über wenigstens 85% der Breite des Flächengebildes erstrecken.

Insbesondere weisen zwei von vier Seiten des Flächengebildes Faltkanten auf, wobei jedoch im Gegensatz zum Stand der Technik, bei dem die Faltkanten auf zwei nebeneinander geordneten Seiten angeordnet sind und über Eck aneinander stoßen, hier die Faltkanten an gegenüberliegenden Seiten angeordnet sind. Diese Anordnung besitzt des Weiteren den Vorteil, dass beim Verschließen der Seitenkanten beispielsweise durch eine thermische Prägeeinrichtung beide zu verschließenden Seitenkanten in eine Richtung weisen, so dass ein Verschließen der Schnittkanten erheblich vereinfacht erfolgen kann. Darüber hinaus besitzen solche Flächengebilde den Vorteil, mit beiden Seiten, Ober- und Unterseite, gleichermaßen auf eine Wunde aufbringbar zu sein.

Darüber hinaus kann auch an den Seiten, die Faltkanten aufweisen, ein Prägen stattfinden, so dass die einzelnen Lagen miteinander verbunden werden.

Insbesondere kann das Flächengebilde aus einer aus Vliesmaterial bestehenden Flachmaterialbahn hergestellt sein, wobei in das Flächengebilde weitere Schichten, wie nachfolgende Funktionsschichten integriert sein können. Vorzugsweise besteht das Flächengebilde ausschließlich aus einem Vliesmaterial.

Die außen liegenden Vliesmateriallage kann darüber hinaus mit einer oder mehreren anderen Lagen verbunden sein, z.B. durch Verkleben, Verschweißen oder Verpressen.

Als Vliesmaterial kann besonders bevorzugt ein hydrophobes Vliesmaterial eingesetzt werden. Dieses hydrophobe Vliesmaterial kann aus einem hydrophoben Vliesstoff oder einem Vliesstoff, der hydrophobe oder hydrophobierte Fasern umfasst, gefertigt sein. Dabei besitzen hydrophobe Vliese den Vorteil, nicht an einer Wunde anzuhaften. Als hydrophobe Vliesmaterialien können insbesondere Vliesstoffe zum Einsatz kommen, die hydrophobe oder hydrophobierte Fasern aus Polyethylen, Polypropylen, Polyamid, Viskose, Polyester oder Mischungen hiervon umfassen.

Es kann aber auch vorgesehen sein, ein hydrophiles Vliesmaterial zu verwenden.

Als Vliesmaterial kann insbesondere ein Kardenvlies oder ein Spinnvlies oder ein Meltblownvlies oder ein wasservernadeltes Vlies oder ein Nadelvlies oder ein Laminat aus zwei oder mehreren gleichen oder verschiedenen der vorgenannten Vliesmaterialien Verwendung finden.

Die Vliesmaterialien können darüber hinaus ihrerseits geprägt oder auf andere Weise zusätzlich verfestigt sein.

Des weiteren kann ein erfindungsgemäßes Flächengebilde mindestens eine Funktionsschicht umfassen, insbesondere kann es sich bei dieser Funktionsschicht um eine geruchsabsorbierenden Schicht, eine antimikrobiell wirksam ausgerüstete Schicht, eine saugende Schicht oder eine einen Superabsorber umfassende Schicht handeln.

Die Erfindung soll im Folgenden anhand eines Beispiels sowie einer Zeichnung näher erläutert werden. Dabei zeigen die

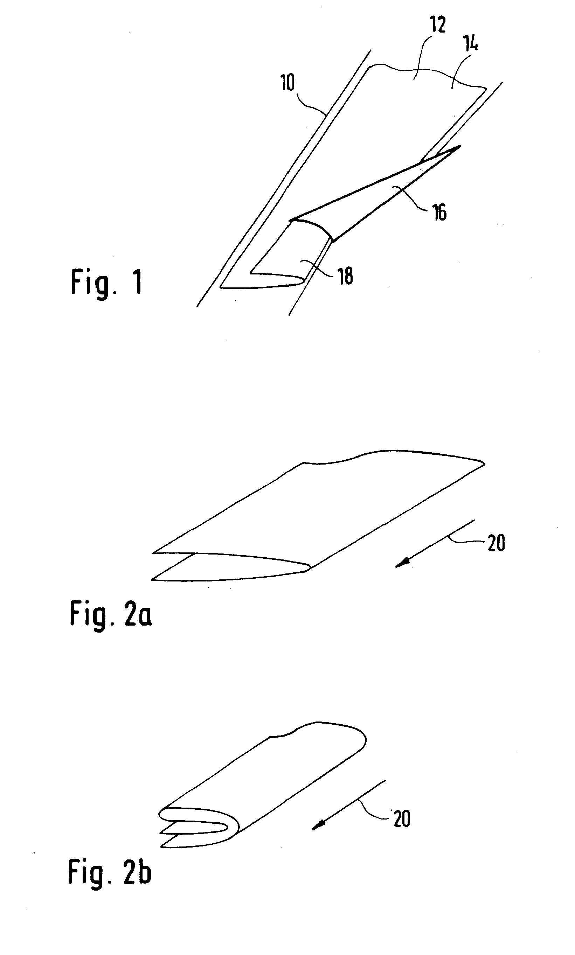

Figuren 1 bis 5 Ausführungsbeispiele der Erfindung.

Figur 1 zeigt in stark schematisierter Darstellung eine Vorrichtung, mit der das Verfahren ausgeführt werden kann, umfassend ein Förderband 10, auf dem das Bahnmaterial, aus dem das Flächengebilde hergestellt werden soll, und das selbst mit 12 bezeichnet ist kontinuierlich oder semikontinuierlich gefördert wird. Dabei besitzt das Bahnmaterial in einem ersten Bereich 14 eine Eingangsbreite von x cm. Das Bahnmaterial 12 wird nun im Bereich einer Umlenkeinrichtung, in der Umlenkbleche 16 vorgesehen sind, derart umgelegt, dass eine Hälfte des Bahnmaterials zur Bildung einer zweiten Lage auf der anderen Hälfte des Bahnmaterials zu liegen kommt, wie es im Bereich 18 des Bahnmaterials 12 gezeigt ist. Das Bahnmaterial besitzt hier eine erste Endbreite von ¹1 x cm.

Beispielsweise kann vorgesehen sein, dass die Breite x 40 cm entspricht. Nach dem ersten Umlegen wäre das Bahnmaterial daherx also 20 cm breit. Nach einem weiteren Umlegeschritt betrüge die Breite des fertigen Produkts 10 cm, wobei dann ein Ablängen derart erfolgt, dass Kompressen mit den Maßen 10x10 cm erhalten werden.

Das so entstandene Material ist in Figur 2a dargestellt, wobei die Maschinenlaufrichtung mit 20 gekennzeichnet ist. Der in Figur 1 gezeigte Schritt wird nun mit dem bereits einmal gefalteten Material, das bereits zweilagig ist, in ebensolcher Weise wiederholt, wobei ein zweites Umlenkblech 16 vorgesehen ist. Das durch den zweiten Schritt erhaltene Material ist dann in Figur 2b dargestellt.

Das in Figur 2b gezeigte Material stellt hier das Endmaterial dar, aus dem nun die Flächengebilde erzeugt werden können, indem das Material abgelängt wird. Die spätere Kompressen kann durch gegebenenfalls Verschließen der Ränder des Materials entstehen.

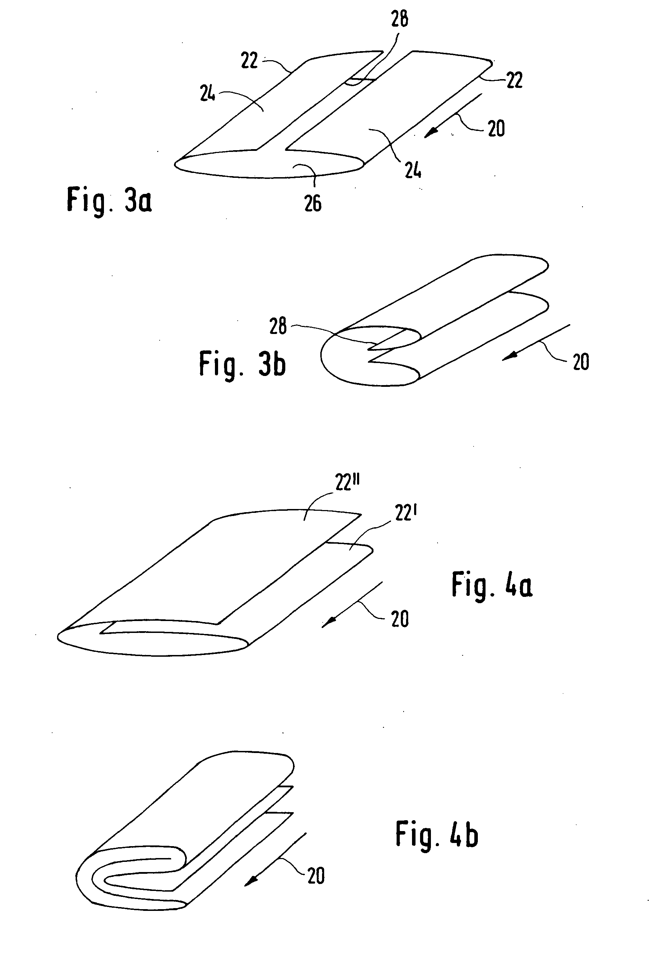

Figur 3 zeigt eine weitere Variante einer Faltung eines vierlagigen Materials für ein Flächengebilde zur Behandlung von Wunden in zwei Schritten. So wird zunächst durch die beidseits der Förderstrecke 10 vorgesehene Umlenkfläche 16, die entweder parallel zueinander angeordnet sein können oder auch hintereinander versetzt, ein Umlegen derart vorgenommen, dass zwei Faltkanten 22, die beide parallel zur Maschinenrichtung 20 verlaufen und beidseits der Längsachse des Bahnmaterials 12 angeordnet sind, entstehen und der jeweils umgelegte Teil 24 im Wesentlichen die Hälfte des nicht umgelegten Teils 26 abdeckt, so dass insgesamt ein zweilagiges Material erzeugt wird. Im zweiten Schritt, der in Figur 3b gezeigt ist, wird die in Figur 3a angeordnete rechte Hälfte auf die linke Hälfte geschlagen, so dass die offenen Kanten 28 des Bahnmaterials im Inneren des fertig gefalteten Gebildes angeordnet sind, so dass die einzigen offenen Kanten die später entstehenden zwei Schnittkanten sind, die durch Schneiden quer zur Maschinenrichtung 20 entstehen.

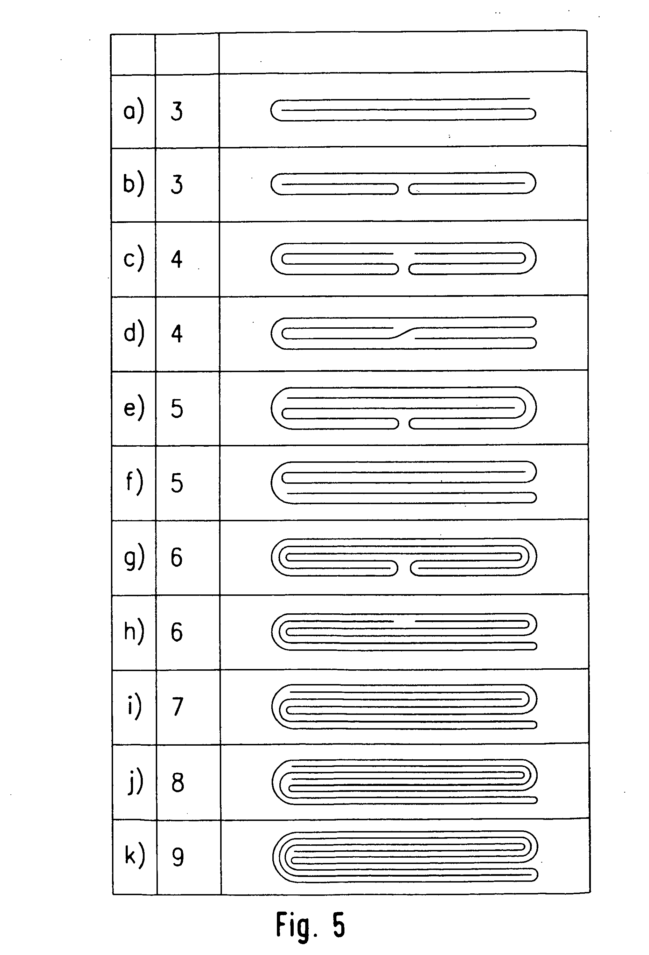

Figur 4 zeigt die Herstellung eines sechslagigen Materials. In dem in Figur 4a dargestellten ersten Schritt wird ein erster Teil, der hier mit 22' gekennzeichnet ist, so umgelegt, dass er die Hälfte des übrigen Bahnmaterials bezüglich der Breite desselben überdeckt. In einem zweiten Schritt wird dann ein Teil 22", der die gleiche Breite wie der Teil 22' aufweist, über den Teil 22' durch ein weiteres Umlenkblech 16 geschlagen. Auf diese Weise entsteht ein dreilagiges Bahnmaterial, das über seine gesamte Breite dreilagig ausgestaltet ist. In einem in Figur 4b gezeigten weiteren Schritt wird dann die in Figur a gezeigte linke Hälfte über die rechte Hälfte mittels eines weiteren Umlenkblechs 16 geschlagen, so dass endgültig ein sechslagiges Produkt entsteht.

Figur 5 zeigt schließlich eine Tabelle, in der verschiedene mögliche Faltvarianten dargestellt sind. Dabei ist in der linken Spalte die Anzahl der Lagen angegeben, die das Produkt besitzt sowie eine Nummerierung. Die rechte Spalte beinhaltet eine schematische Darstellung eines Querschnitts durch ein entsprechendes Produkt, also eines Schnitts quer zur Maschinenrichtung. Das vorstehend bereits bezeichnete Produkt, wie es in Figur 4a dargestellt ist, nämlich ein dreilagiges Produkt a) ist erfindungsgemäß und kann zu Kompressen verarbeitet werden.

Die erfindungsgemäßen Gestaltungen gemäß den Darstellungen b), c), e) und g) sind weniger bevorzugt, da hier, wie gut erkannt werden kann, eine Ober- bzw. Unterseite nicht durchgehend gestaltet ist, wobei die untere Lage durch zwei umgelegte Teile gebildet ist, die aufeinander zuweisen. In diesen Ausführungsformen erstreckt sich die untere Lage nicht über die volle Breite des Flächengebildes jedoch über mehr als 85 % dieser Breite. Diese Produkte lassen sich nicht so gut zweiseitig einsetzen. Die beiden Kanten, die an einer äußeren Lage aufeinandertreffen, werden in der Regel entweder miteinander oder mit der darunter liegenden Lage verbunden, um sie so festzulegen.

Den übrigen Darstellungen können bevorzugte erfindungsgemäße Produkte entnommen werden, die zwischen drei und neun Lagen aufweisen. Grundsätzlich sind weitere Lagen denkbar. Dabei sind die Gestaltungen mit vier oder mehr Lagen in der dargestellten Form besonders vorteilhaft, da von den ursprünglichen Längskanten des Bahnmaterials keine Kante außen liegend ist, sondern sämtliche Kanten im Inneren des gefalteten Flächengebildes angeordnet sind, so dass es hier nicht zu einem Wundkontakt innen liegender Schichten des Bahnmaterials kommen kann, sondern vielmehr geschlossene Oberflächen allseitig gegeben sind. Auf diese Weise können insbesondere aktive Materialien, wie Superabsorber oder Aktivkohle, sowie weitere Zugaben sicher im Inneren des Wundversorgungsmaterials bereitgestellt und fixiert werden, ohne dass die Gefahr besteht, dass das Material mit der Wunde in Kontakt kommt.

## Patentansprüche

1. Verfahren zum Herstellen von Flächengebilden aus Vliesmaterial zur Wundversorgung, insbesondere Kompressen, bei dem ein erster Teil einer Flachmaterialbahn zunächst entlang einer parallel zu einer Maschinenrichtung verlaufenden ersten Faltkante so umgelegt wird, dass der erste Teil der Flachmaterialbahn den übrigen anderen Teil zumindest teilweise zur Bildung einer zweiten Lage überdeckt, **dadurch gekennzeichnet, dass** anschließend mindestens ein weiteres Mal ein weiterer Teil der Flachmaterialbahn entlang einer parallel zu der Maschinenrichtung verlaufenden weiteren Faltkante so umgelegt wird, dass der weitere Teil wiederum auf dem übrigen anderen Teil der Flachmaterialbahn zu liegen kommt, unter Ausbildung eines zumindest teilweise wenigstens dreilagigen Flächengebildes, wobei sämtliche Faltkanten des erhaltenen Flächengebildes parallel zur Maschinenrichtung verlaufen und das Flächengebilde eine die Ober- sowie eine die Unterseite bildende Lage aufweist, wobei sich diese Lagen wenigstens über 85% der Breite des Flächengebildes erstrecken.

2. Verfahren nach Anspruch 1, **dadurch gekennzeichnet, dass** der weitere Teil, der in einer weiteren Faltung umgelegt wird, einlagig oder mehrlagig ist.

3. Verfahren nach Anspruch 1 oder 2, **dadurch gekennzeichnet, dass** das Flächengebilde wenigstens teilweise vier oder mehr als vier Lagen aufweist.

4. Verfahren nach einem der vorangehenden Ansprüche, **dadurch gekennzeichnet, dass** die Lagen, die die Ober- und die Unterseite des Flächengebildes bilden, sich über wenigstens 900, insbesondere über wenigstens 95% sowie insbesondere über 100% der Breite des Flächengebildes nach dem Falten erstrecken.

5. Verfahren nach einem der vorangehenden Ansprüche, **dadurch gekennzeichnet, dass** der jeweils umgelegte Teil den übrigen Teil um mindestens ein Viertel, insbesondere ein Drittel, sowie vorzugsweise mindestens die Hälfte oder insbesondere ganz überdeckt.

6. Verfahren nach einem der vorangehenden Ansprüche, **dadurch gekennzeichnet, dass** das Vliesmaterials herstellungsbedingt eine Quer- sowie eine Vliesmaschinenrichtung aufweist und die Faltkanten parallel zur Vliesmaschinenrichtung angeordnet werden.

7. Verfahren nach einem der vorangehenden Ansprüche, **dadurch gekennzeichnet, dass** die Ober- und/oder Unterseite des Flächengebildes durch Lagen gebildet werden, die einstückig bzw. durchgehend ausgebildet sind.

8. Verfahren nach einem der vorangehenden Ansprüche, **dadurch gekennzeichnet, dass** die Flächengebilde quer zur Richtung der Faltkanten abgelängt werden.

9. Verfahren nach Anspruch 8, **dadurch gekennzeichnet, dass** die beim Ablängen entstehenden Schnittkanten verschlossen und/oder verfestigt werden.

10. Verfahren nach einem der vorangehenden Ansprüche, **dadurch gekennzeichnet, dass** die Flachmaterialbahn aus einem ein- oder mehrschichtigen Material und/oder Laminat besteht.

11. Verfahren nach einem der vorangehenden Ansprüche, **dadurch gekennzeichnet, dass** eine oder mehrere der Schichten als Funktionsschichten ausgebildet sind, wobei sich die Funktionsschichten über die gesamte Breite der Flachmaterialbahn oder lediglich über einen Teil hiervon erstrecken.

12. Flächengebilde zur Wundversorgung umfassend ein Vliesmaterial, insbesondere hergestellt nach dem vorstehenden Verfahren, bei dem sämtliche Faltkanten parallel verlaufen und das zumindest teilweise wenigstens dreilagig ausgebildet ist, wobei sich die eine Ober- und eine Unterseite bildenden Lagen über wenigstens 85% der Breite des Flächengebildes erstrecken.

13. Flächengebilde nach Anspruch 12, **dadurch gekennzeichnet, dass** zwei von vier Seitenkanten Faltkanten aufweisen, **dadurch gekennzeichnet, dass** die Faltkanten an gegenüberliegenden Seitenkanten des Flächengebildes angeordnet sind.

14. Flächengebilde nach Anspruch 13, **dadurch gekennzeichnet, dass** es eine Kompresse ist.
